# EUROPEAN PATENT APPLICATION

(11) **EP 3 181 130 A1**
(43) Date of publication of application: **21.06.2017**
(21) Application number: 14899917.0
(22) Date of filing: 12.08.2014
(51) Int. Cl.: A61K 31/192, A61K 31/216, A61K 31/353, A61P 1/02, A61P 17/00, A61P 17/02, A61P 31/02, A61P 39/02, A61P 43/00

(54) **CELL REPAIR AGENT AND DISINFECTION SYSTEM**

(71) Applicant: Kanno, Minoru, Sendai-shi, Miyagi 980-0871 (JP)
(72) Inventor: NIWANO, Yoshimi, Sendai-shi Miyagi 980-8577 (JP); KANNO, Taro, Sendai-shi Miyagi 980-8577 (JP)
(74) Representative: Plougmann Vingtoft a/s
(86) International application number: PCT/JP2014/071330
(87) International publication number: WO 2016/024339

(57) **Abstract**

To provide a cell repair agent capable of repairing cells damaged by the oxidizing power of acidic electrolyzed water or the like, and, a disinfection system capable of safely and efficiently accelerating the sterilization and cure of infected skin or intraoral wounds. The cell repair agent contains at least one type of a polyphenol represented by the following general formula (1), a polymer thereof, and a pharmaceutically acceptable salt thereof R₂ and R₃ are preferably hydrogen atoms, and R₁ is preferably a hydrogen atom or a hydroxyl group.

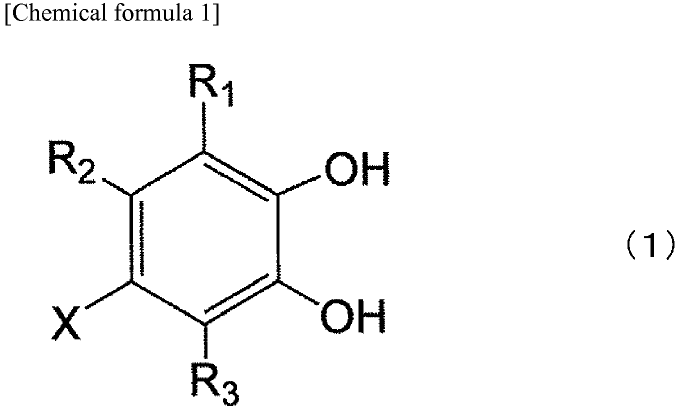

## Description

### TECHNICAL FIELD

The present invention relates to a cell repair agent and a disinfection system.

### BACKGROUND ART

The risk of infectious diseases increases with age. For age groups of 60 or older in Japan, an infectious disease, pneumonia, is the fourth leading cause of death. Infectious diseases include various pathological conditions ranging from life-threatening diseases such as pneumonia and septicemia to skin infections such as dermatophytosis (vesicular eczema) and dental diseases such as periodontal disease, which differ in causative microorganisms or infected sites, for example. Removal of pathogenic microbes is the primary objective of treatment for all of these infectious diseases, and a drug such as an antibiotic is administered for treatment in many cases. However, the widespread use of antibiotics results in the advent of drug-resistant organisms, and this may soon lead to severe problems such as the development of hospital-acquired infections due to multidrug-resistant bacteria. Therefore, the establishment of sterilizing therapy as an alternative to antibiotic administration is desired.

Acidic electrolyzed water that is obtained by electrolysis of sodium chloride or potassium chloride solution contains hypochlorous acid as the main contributor to the activity (for example, see Non-patent Document 1). When hypochlorous acid enters cells, hydroxyl radicals, which are a type of active oxygen, are generated and exert a sterilizing effect because of the strong oxidizing power (for example, see Non-patent Document 2). Moreover, a disinfection technology based on photolysis of hydrogen peroxide involves the application of the oxidizing power of hydroxyl radicals generated by homolytic cleavage of hydrogen peroxide resulting from irradiation of hydrogen peroxide with near-ultraviolet visible light (for example, see Non-patent Document 3).

A big advantage of sterilization technology using hydroxyl radicals, such as acidic electrolyzed water or disinfection technology based on photolysis of hydrogen peroxide over conventional sterilizing therapies involving antibiotic administration is to allow no development of resistant bacteria (for example, see Non-patent Document 4). Acidic electrolyzed water is used not only in the field of foods, but also in the medical field because of this advantage. In particular, the use of acidic electrolyzed water is being attempted for disinfection required for oral infectious diseases or as sterilization for surgeries (for example, see Non-patent Document 4 or 5). Applying the photosterilization technology using hydrogen peroxide to periodontal diseases is being explored (for example, see Non-patent Document 6). However, there is a concern that both acidic electrolyzed water and the disinfection technology based on photolysis of hydrogen peroxide can damage not only pathogenic microbes, but also cells at the disease site, because of the strong oxidizing power (for example, see Non-patent Documents 7 to 9). Furthermore, some existing non-hydroxy-radical-mediated disinfectants possess strong cytotoxicity and there is a similar concern that such disinfectants damage cells at the disease site (for example, see Non-patent Document 10).

On the other hand, polyphenols have an antioxidant effect and are known to have an effect of inhibiting oxidative injuries of local tissues due to active oxygen produced by inflammatory cells such as neutrophils at inflamed sites. Polyphenols are used for skin preparations, oral agents, and the like (for example, see Patent Documents 1 to 6, Non-patent Document 11 or 12).

### PRIOR ART DOCUMENTS

### NON-PATENT DOCUMENTS

Non-patent Document 1: T. Mokudai et al., "Presence of hydrogen peroxide, a source of hydroxyl radicals, in acid electrolyzed water", PLoS ONE, 2012, 7, e46392
Non-patent Document 2: Satoshi Fukuzaki, "Mechanisms of Actions of Hypochlorous Acid as Cleaning and Disinfecting Agents in Relation to Injury to Bacteria", Japanese Journal of Food Microbiology, 2009, 26, p.76-80
Non-patent Document 3: H. Ikai et al., "Photolysis of hydrogen peroxide, an effective disinfection system via hydroxyl radical formation", Antimicrobial Agents and Chemotherapy, 2010, 49, p.5086-5091
Non-patent Document 4: H. Ikai et al., "In vitro evaluation of the risk of inducing bacterial resistance to disinfection treatment with photolysis of hydrogen peroxide", PLoS ONE, 2013, 8, e81316
Non-patent Document 5: Noriaki Tanaka et al., "Dialysis care and strongly acidic electrolyzed water", Nihon Toseki Igakkai Zasshi, 2004, 37, p.1273-1283
Non-patent Document 6: A. Oyamada et al., "In vitro bactericidal activity of photo-irradiated oxydol products via hydroxyl radical generation", Biocontrol Sciences, 2013, 18, p.88
Non-patent Document 7: K. Okubo et al., "Cytotoxicity and microbicidal activity of electrolyzed strong acid water and acidic hypochlorite solution under isotonic conditions", The Journal of the Japanese Association for Infectious Diseases, 1999, 73, p.1025-1031
Non-patent Document 8: K. Gomi et al., "Microbicidal and cytotoxic effects of functional water in vitro", Quintessence International, 2010, 41, e166-172
Non-patent Document 9: Hiroyo Ikai et al., "Evaluation of cytotoxicity of hydroxyl radicals generated by photolysis of hydrogen peroxide", The 64th Meeting of Society for Free Radical Research JAPAN -Program·Abstracts-, page 73.
Non-patent Document 10: Keiji Nakajima et al., "Inhibitory effect of disinfectant on cell proliferation and wound healing (in vitro)", Journal of Japanese Society of Hospital Pharmacists, 2004, 40, p.1559-1562.
Non-patent Document 11: M. A. Soobrattee et al., "Phenolics as potential antioxidant therapeutic agents: mechanism and actions", Mutation Research, 2005, 579, p.200-213
Non-patent Document 12: C. A. RICE-EVANS et al., "Structure-antioxidant activity relationships of flavonoids and phenolic acids", Free Radical Biology & Medicine, 1996, 20, p.933-956

### PATENT DOCUMENTS

Patent Document 1: International Publication WO2005/092327
Patent Document 2: JP Patent Publication (Kokai) No. 06-336422 A (1994)
Patent Document 3: JP Patent No. 3917370
Patent Document 4: JP Patent Application No. 2005-213234
Patent Document 5: JP Patent Publication (Kokai) No. 2009-52185 A
Patent Document 6: JP Patent Publication (Kohyo) No. 2010-511026 A

### SUMMARY OF THE INVENTION

### PROBLEMS TO BE SOLVED BY THE INVENTION

As described in Non-patent Documents 7 to 10, there is a concern that acidic electrolyzed water, the disinfection technology based on photolysis of hydrogen peroxide, and existing disinfectants may damage not only pathogenic microbes, but also cells at disease sites because of the strong oxidizing power, and the development of technology for repairing the resulting damage has been expected. Furthermore, as described in Patent Documents 1 to 6 and Non-patent Documents 11 and 12, polyphenols are known to have an effect of inhibiting oxidative damage to local tissues due to active oxygen produced by inflammatory cells. However, it has not been confirmed that polyphenols have an effect of repairing cells severely damaged by acidic electrolyzed water or the like.

The present invention has been achieved, noting these problems. An objective of the present invention is to provide a cell repair agent capable of repairing cells damaged by the oxidizing power of acidic electrolyzed water or the like, and, a disinfection system capable of safely and efficiently accelerating the sterilization and cure of infected skin or intraoral wounds.

### SOLUTIONS TO THE PROBLEMS

As a result of intensive studies to achieve the above objective, the present inventors have discovered that a specific polyphenol having a catechol group or a pyrogallol group and a polymer composition thereof have an effect of repairing cells severely damaged by acidic electrolyzed water, and thus have completed the present invention.

Specifically, the cell repair agent according to the present invention contains at least one type of a polyphenol represented by the following general formula (1), a polymer thereof, and a pharmaceutically acceptable salt thereof 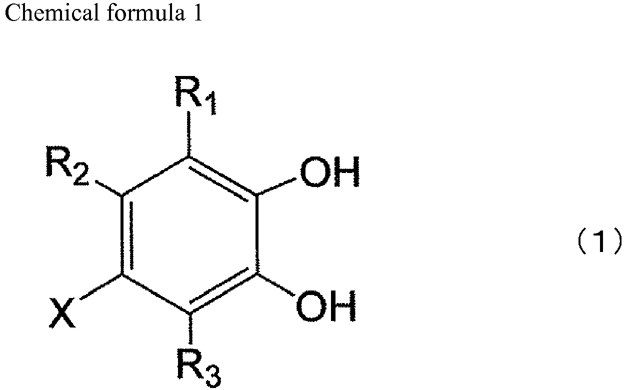 (wherein R₁ to R₃ are each selected from a group consisting of a substituted or unsubstituted hydrogen atom, a hydroxyl group, a halogen atom, a nitro group, a cyano group, a thio group, an alkoxy group, an aryloxy group, a heteroaryloxy group, a carbonyl group, an amino group, a C₁₋₁₀ alkylamino group, a sulfonamide group, an imino group, a sulfonyl group, a sulfinyl group, a C₁₋₁₀ alkyl group, a C₃₋₁₂ cycloalkyl group, a C₃₋₁₂ heterocycloalkyl group, a C₉₋₁₂ bicycloalkyl group, a C₃₋₁₂ heterobicycloalkyl group, a C₁₋₁₀ arylalkyl group, a C₁₋₅ heteroarylalkyl group, a C₁₋₁₀ parhaloalkyl group, a C₁₋₃ carbonylalkyl group, a C₁₋₃ thiocarbonylalkyl group, a C₁₋₃ sulfonylalkyl group, a C₁₋₃ sulfinylalkyl group, a C₁₋₁₀ aminoalkyl group, a C₁₋₃ iminoalkyl group, an aryl group, a heteroaryl group, a C₉₋₁₂ bicycloaryl group, and a C₄₋₁₂ hetero bicycloaryl, are each independent, or, any two of R₁, R₂, and R₃ together form a substituted or unsubstituted ring, and X is a substituted or unsubstituted C₁₋₂₀ aliphatic hydrocarbon group, a C₃₋₂₀ aromatic hydrocarbon group, or a C₃₋₂₀ polycyclic aromatic hydrocarbon group.)

Specifically, the cell repair agent according to the present invention contains at least one type of a polyphenol represented by the following general formula (1), a polymer thereof, and a pharmaceutically acceptable salt thereof, so as to be able to repair cells damaged by the oxidizing power of acidic electrolyzed water or the like to be used for disinfection. The cell repair agent can also be expected to have an effect of inhibiting the oxidative damage to local tissues due to active oxygen produced by inflammatory cells such as neutrophils at inflamed sites.

In the cell repair agent according to the present invention, the above R₂ and the above R₃ are preferably hydrogen atoms. In addition, the above R₁ is preferably a hydrogen atom or a hydroxyl group. Especially in such a case, the cell repair agent has a high effect of repairing severely damaged cells.

In the cell repair agent according to the present invention, the above polyphenol is preferably selected from a group consisting of proanthocyanidin, (+)-catechin, chlorogenic acid, caffeic acid and gallic acid. In this case, the cell repair agent can also repair cells damaged by the oxidizing power of acidic electrolyzed water or the like to be used for disinfection.

The disinfection system according to the present invention comprises a disinfection means for performing sterilization and/or disinfection for a living body, and a post-treatment means mounted to be able to bring the cell repair agent according to the present invention into contact with a part of said living body to be treated by the above disinfection means.

The disinfection system according to the present invention is capable of repairing cells damaged by the oxidizing power of acidic electrolyzed water or the like used for disinfection by treating the cells with the cell repair agent according to the present invention after disinfection. Therefore, the disinfection system according to the present invention can safely and efficiently accelerate the sterilization and cure of infected skin or intraoral wounds. Moreover, the disinfection system according to the present invention is easy to handle and capable of safely and efficiently performing disinfection of infected sites, surgeries, and the like even via short-term treatment. Examples of a disinfection means include an apparatus for injecting, spraying, immersing, draining, or applying a liquid microbicide and/or disinfectant, and an apparatus for filling with or injecting a gas microbicide and/or disinfectant. Examples of a post-treatment means include an apparatus for injecting, spraying, immersing, draining, or applying a solution or a dispersed solution of a cell repair agent. Such a post-treatment means may be designed to operate independently from a disinfection means, or designed to operate in conjunction with a disinfection means for contacting with a cell repair agent, when the disinfection means is operated.

### EFFECTS OF THE INVENTION

According to the present invention, a cell repair agent capable of repairing cells damaged by the oxidizing power of acidic electrolyzed water or the like, and, a disinfection system capable of safely and efficiently accelerating the sterilization and cure of infected skin or intraoral wounds can be provided.

### BRIEF DESCRIPTION OF THE DRAWINGS

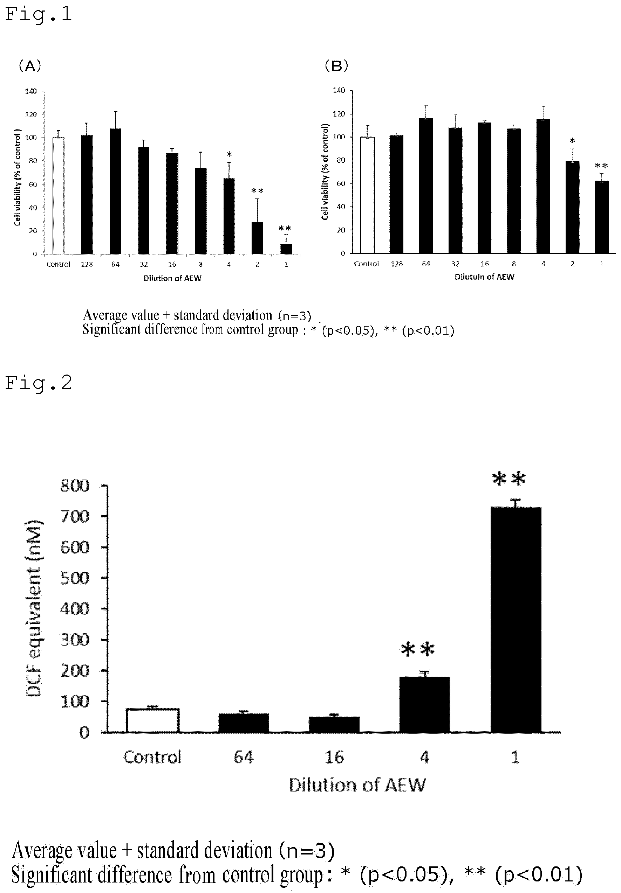

Fig. 1 shows graphs relating to the cell repair agents of the embodiments of the present invention and that show the impact of various dilution ratios (dilutions) of acidic electrolyzed water (AEW) on (A) the cell proliferation rate (cell viability) of mouse fibroblasts in growth phase, and (B) the cell proliferation rate (cell viability) of mouse fibroblasts in stationary phase.
Fig. 2 is a graph relating to the cell repair agents of the embodiments of the present invention and that shows the levels of ROS generated within mouse fibroblasts in stationary phase resulting from various dilution ratios (dilutions) of acidic electrolyzed water (AEW).

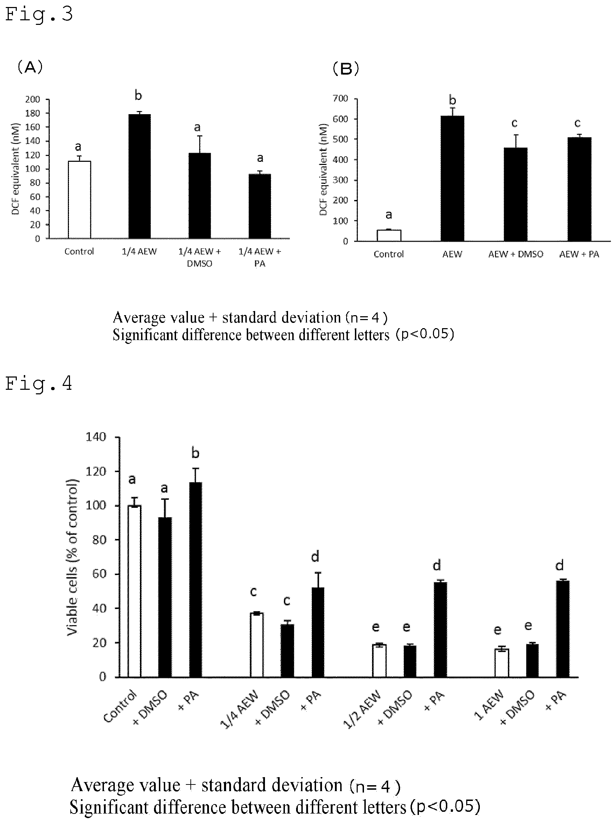

Fig. 3 shows graphs relating to the cell repair agents of the embodiments of the present invention, and that show intracellular ROS levels when the intracellular ROS levels of mouse fibroblasts were increased by (A) four-fold-diluted acidic electrolyzed water, or (B) undiluted acidic electrolyzed water, followed by treatment with dimethyl sulfoxide (DMSO) or proanthocyanidin (PA).
Fig. 4 is a graph relating to the cell repair agents of the embodiments of the present invention and that shows the degree of cell proliferation (viable cells) when mouse fibroblasts in growth phase were treated with various dilutions of acidic electrolyzed water, followed by post-treatment with DMSO or proanthocyanidin.

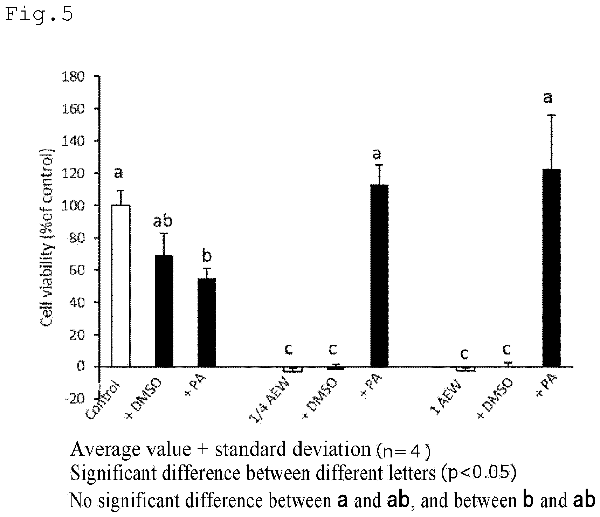

Fig. 5 is a graph relating to the cell repair agents of the embodiments of the present invention and that shows the degree of cell proliferation (cell viability) when human gingival fibroblasts in growth phase were treated with various dilutions of acidic electrolyzed water, followed by post-treatment with DMSO or proanthocyanidin.

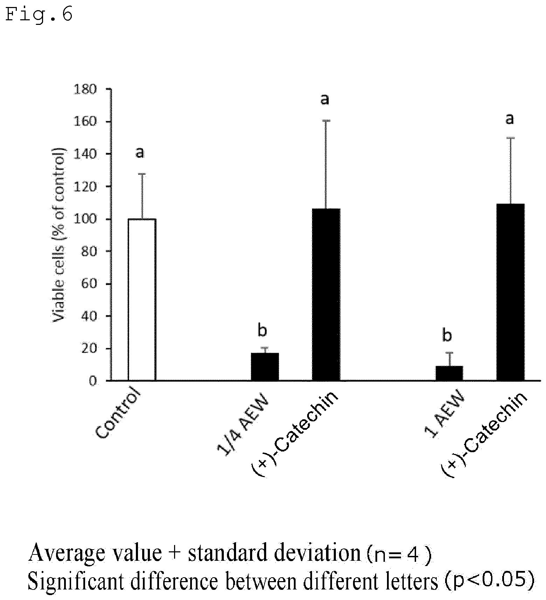

Fig. 6 is a graph relating to the cell repair agents of the embodiments of the present invention and that shows the degree of cell proliferation (viable cells) when mouse fibroblasts in growth phase were treated with a 1/4 dilution of acidic electrolyzed water and undiluted acidic electrolyzed water, followed by post-treatment with (+)-catechin.

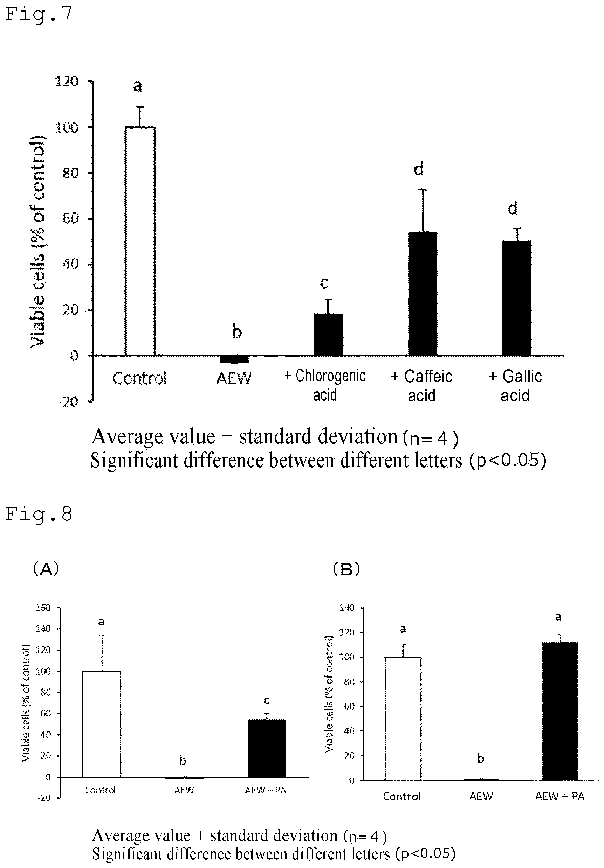

Fig. 7 is a graph relating to the cell repair agents of the embodiments of the present invention and that shows the degree of cell proliferation (viable cells) when mouse fibroblasts in growth phase were treated with undiluted acidic electrolyzed water, followed by post-treatment with chlorogenic acid, caffeic acid, or gallic acid.
Fig. 8 shows graphs relating to the cell repair agents of the embodiments of the present invention and that show the degree of cell proliferation (viable cells) when mouse fibroblasts in growth phase were treated with undiluted acidic electrolyzed water, followed by post-treatment with proanthocyanidin for (A) 1 minute or (B) 5 minutes.

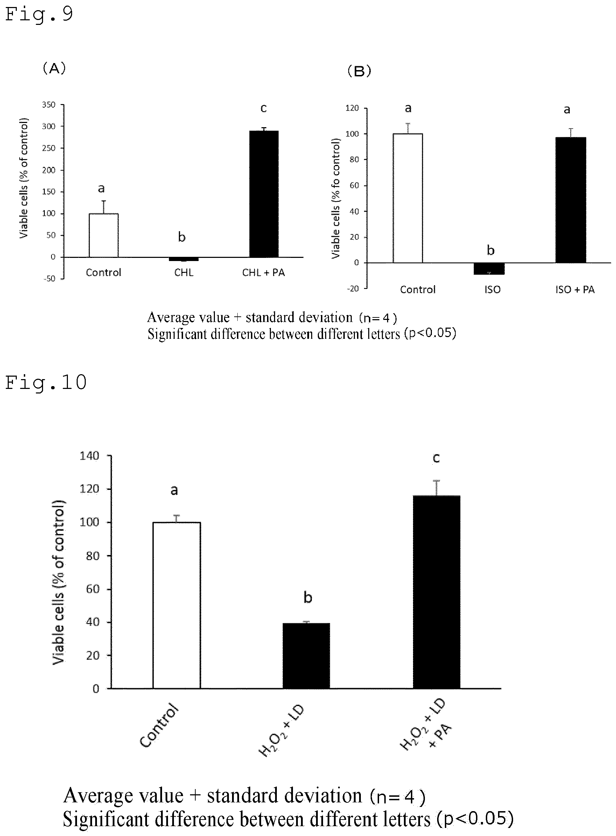

Fig. 9 shows graphs relating to the cell repair agents of the embodiments of the present invention and that show the degree of cell proliferation (viable cells) when mouse fibroblasts in growth phase were treated with (A) undiluted chlorhexidine solution (CHL) or (B) 15-fold-diluted iodine solution (ISO), followed by post-treatment with proanthocyanidin.
Fig. 10 is a graph relating to the cell repair agents of the embodiments of the present invention and that shows the degree of cell proliferation (viable cells) when human gingival fibroblasts in growth phase were treated by a disinfection method based on photolysis of hydrogen peroxide (H₂O₂+LD), followed by post-treatment with proanthocyanidin.

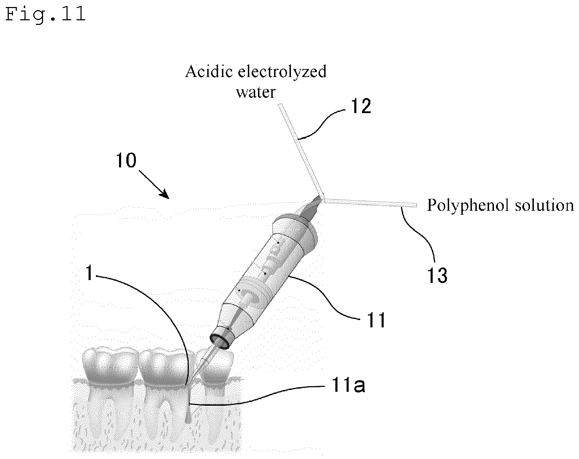

Fig. 11 is a perspective view showing an example of the application of the disinfection system of an embodiment of the present invention to a periodontal infection.

### EMBODIMENTS OF THE INVENTION

The cell repair agent of each embodiment of the present invention contains at least one type of a polyphenol represented by the above general formula (1), a polymer thereof, and a pharmaceutically acceptable salt thereof

The cell repair agents of the embodiments of the present invention will be explained as follows based on the results of various tests. In addition, the present invention is not limited by these test examples (Examples).

### Test 1: Impact of acidic electrolyzed water on mouse fibroblasts in growth phase and mouse fibroblasts in stationary phase

A test was conducted for examining the impact of acidic electrolyzed water on mouse fibroblasts in growth phase and mouse fibroblasts in stationary phase. Acidic electrolyzed water (AEW) to be used in this test was obtained by 15 minutes of electrolysis of a 0.06% to 0.08% aqueous sodium chloride solution as an electrolyte solution and an electrolysis apparatus ("ALTRON-MINI AL-700", ALTEC Co., Ltd.), alternating voltage of 100V, and rated current of 0.6A. The thus obtained acidic electrolyzed water was measured for pH, oxidation-reduction potential (ORP) and residual chlorine concentration using a pH/ORP meter ("SG2", Mettler-Toled) and a residual chlorine meter ("HI96771C", HANNA Instruments Japan). As a result, it was confirmed for acidic electrolyzed water that the pH ranged from 2.2 to 2.7, the ORP was 1100 mV or more, and the residual chlorine concentration ranged from 30 mg/l to 60 mg/l.

The test was conducted as follows. First, cells (mouse-derived 3T3-L1 fibroblasts; purchased from DS Pharma Biomedical Co., Ltd.) cultured in a 25-cm² flask under conditions of 37°C and 5%CO₂ were treated with a 0.25% Trypsin-EDTA solution (Life Technologies), and then cell suspensions were prepared according to a standard technique in medium to a cell density of 2x10⁴ cells/ml. Media used herein as growth media were prepared by adding fetal bovine serum (Life Technologies) and a penicillin/streptomycin solution (10000 units/ml penicillin, and 10 mg/ml streptomycin, Wako Pure Chemical Industries, Ltd.) to Dulbecco's Modified Eagle Medium (DMEM, Life Technologies) to 10% (v/v) and 1% (v/v), respectively.

The cell suspension was seeded, 100 µl each, to each well of a 96-well microplate. After cells in growth phase were cultured for about 25 hours and cells in stationary phase were cultured for 4 days under conditions of 37°C and 5%CO₂, the medium was removed. After washing with phosphate-buffered saline (PBS, pH7.4), 100 µl each of two-fold dilution series of acidic electrolyzed water obtained by dilution with pure water was added to each well. After 30 seconds, two-fold dilution series of acidic electrolyzed water was removed, followed by washing with PBS.

Fresh medium was added and culture of cells in growth phase was resumed under conditions of 37°C and 5%CO₂. After 24 hours of culture, the degree of cell proliferation was determined by a MTT (methyl thiazolyl tetrazorium) method. Specifically, after 22 hours of culture, 10 µl each of a MTT reagent (Trevigen) was added to each well, followed by another 2 hours of culture under conditions of 37°C and 5%CO₂. After culture, 100 µl each of a Detergent reagent (Trevigen) was added, cells were left to stand overnight with protection from light at room temperature. Subsequently, the absorbance was read at 595 nm using a microplate reader ("FilterMax™ F5 Multi-Mode Microplate Reader", Molecular Devices). A group treated with sterilized physiological saline was designated as a control group (control). The proportion (%) of each treated group was calculated with the absorbance of the control group designated as 100%.

Cells in stationary phase were washed, 10 µl each of a MTT reagent (Trevigen) was added to each well, and then cells were further cultured for 2 hours under conditions of 37°C and 5%CO₂, followed by similar procedures. The proportion (%) of each treated group was calculated with the absorbance of a control group (control) designated as 100%. In addition, a significant difference to the average value of each control group was tested by Dunnett's multiple comparison test (multiple comparative analysis).

Test results are shown in Fig. 1. As shown in Fig. 1(a), it was confirmed that, up to about a 32-fold dilution of acidic electrolyzed water (AEW), the cell proliferation rate (cell viability) of mouse fibroblasts in growth phase decreased, as the dilution ratio (dilution) of acidic electrolyzed water decreased (as the concentration increased). The cell proliferation rate was significantly low in the case of a maximum four-fold dilution of AEW, suggesting that many cells were damaged by acidic electrolyzed water. As shown in Fig. 1(b), the cell proliferation rate of mouse fibroblasts in stationary phase was confirmed to be significantly low in the case of a maximum two-fold dilution of acidic electrolyzed water (AEW), suggesting that many cells were damaged by acidic electrolyzed water.

### Test 2: Impact of acidic electrolyzed water on mouse fibroblasts in stationary phase -Intracellular ROS levels-

A test was conducted for examining the intracellular levels of reactive oxygen species (ROS) generated when acidic electrolyzed water is brought into contact with mouse fibroblasts in stationary phase. First, mouse fibroblasts in stationary phase prepared and cultured similarly to Test 1 were washed with phosphate-buffered saline, and then 100 µl each of four-fold dilution series of acidic electrolyzed water obtained by dilution with pure water was added to each well. 30 seconds later, two-fold dilution series of acidic electrolyzed water was removed. Immediately after washing with PBS, intracellular ROS levels were measured using a measurement kit ("Oxiselect™ Intracellular ROS Assay Kit", Cell Biolabs).

Specifically, 100 µl of 1 mM 2',7'-dichlorodihydrofluorescin Diacetate (DCFH-DA) dissolved in serum-free medium (DMEM only) was added to each well, and then cells were cultured for 1 hour under conditions of 37°C and 5%CO₂. After culture, washing with phosphate-buffered saline was performed, 100 µl of 2X Cell Lysis Buffer (Cell Biolabs) and 100 µl of serum-free DMEM were added, and then the resultants were left to stand for 5 minutes. 150 µl of the resultants was transferred from each well to a black 96-well microplate, fluorescence intensities were measured at an excitation wavelength of 450 nm and a fluorescence wavelength of 530 nm using a microplate reader ("FilterMax™ F5 Multi-Mode Microplate Reader", Molecular Devices). A standard curve was prepared using 2',7'-dichlorodihydrofluorescein (DCF), and intracellular ROS levels were represented by DCF equivalents (nM). In addition, a significant difference to the average value of each control group (control) was tested by Dunnett's multiple comparison test (multiple comparative analysis).

Test results are shown in Fig. 2. As shown in Fig. 2, the DCF equivalent of mouse fibroblasts in stationary phase significantly increased in the case of a maximum four-fold dilution of acidic electrolyzed water (AEW), confirming increased levels of intracellular reactive oxygen species (ROS). Taken together with the results in Fig. 1, it is considered that when up to two- to four-fold dilutions of acidic electrolyzed water were brought into contact with mouse fibroblasts, intracellular ROS levels increased to induce cytotoxicity, so as to damage many cells.

### Test 3: Impact of DMSO and proanthocyanidin on ROS levels within mouse fibroblasts in stationary phase increased by acidic electrolyzed water

Similarly to Test 2, mouse fibroblasts in stationary phase were treated with four-fold-diluted acidic electrolyzed water and undiluted acidic electrolyzed water for 30 seconds. After washing with PBS, 100 µl each of 140 mM DMSO (Wako Pure Chemical Industries, Ltd.) or 1 mM DCFH-DA containing 1 mg/ml proanthocyanidin ("Leucoselect (Registered Trademark)", Indeda) as a hydroxyl radical scavenger was added to each well, followed by 1 hour of culture under conditions of 37°C and 5%CO₂. After culture, similarly to Test 2, intracellular ROS levels were determined. In addition, a significant difference between groups was tested by Tukey-Krammer's multiple comparison test (multiple comparative analysis).

Test results are shown in Fig. 3. As shown in Fig. 3, it was confirmed that the ROS level in mouse fibroblasts in stationary phase, which had been increased by acidic electrolyzed water (AEW), was significantly decreased by hydroxyradical scavenging agents DMSO and proanthocyanidin (PA).

### Test 4: Impact of DMSO and proanthocyanidin on the cytotoxicity of acidic electrolyzed water against mouse fibroblasts in growth phase

Similarly to Test 1, mouse fibroblasts in growth phase were treated with four-fold-diluted acidic electrolyzed water and undiluted acidic electrolyzed water for 30 seconds. After washing with phosphate-buffered saline, 100 µl of 140 mM DMSO or 1 mg/ml proanthocyanidin prepared with serum-free DMEM was added to each well, and then cells were cultured for 30 minutes under conditions of 37°C and 5%CO₂. After washing with PBS, fresh medium was added, and then the degree of cell proliferation after 24 hours of culture under conditions of 37°C and 5%CO₂ was determined by the MTT method. In addition, a significant difference between groups was tested by Tukey-Krammer's multiple comparison test (multiple comparative analysis).

Test results are shown in Fig. 4. As shown in Fig. 4, no change was confirmed in the cell proliferation state of mouse fibroblasts in growth phase affected by acidic electrolyzed water (AEW), even when DMSO was used. It is thus considered that cells are severely damaged immediately after treatment with acidic electrolyzed water because of cytotoxicity induced against the cells by ROS levels increased by acidic electrolyzed water, and thus the cells cannot recover from damage even when ROS is eliminated using DMSO. In contrast, the cell proliferation state was confirmed to be significantly improved when proanthocyanidin was used, suggesting that the cells were repaired and recovered from the damage.

### Test 5: Impact of DMSO and proanthocyanidin on the cytotoxicity of acidic electrolyzed water against human gingival fibroblasts in growth phase

A test similar to Test 4 was conducted for human gingival fibroblasts in growth phase. First, human gingival fibroblasts (purchased from Primary Cell Co., Ltd.) were cultured in a 25 cm² flask under conditions of 37°C and 5%CO₂, and then a cell suspension was prepared similarly to the case of mouse fibroblasts in medium to a cell density of 2x10⁴ cells/ml. 100 µl each of the cell suspension was seeded to each well of a 96-well microplate. After 24 hours of culture under conditions of 37°C and 5%CO₂, similarly to Test 4, cells in growth phase were treated with four-fold-diluted acidic electrolyzed water or undiluted acidic electrolyzed water for 30 seconds. After washing with PBS, 100 µl of 140 mM DMSO or 1 mg/ml proanthocyanidin prepared with serum-free DMEM was added to each well, followed by 30 minutes of culture under conditions of 37°C and 5%CO₂. After washing with PBS, fresh medium was added, and then the degree of cell proliferation after 24 hours of culture under conditions of 37°C and 5%CO₂ was determined by the MTT method. In addition, a significant difference between groups was tested by Tukey-Krammer's multiple comparison test (multiple comparative analysis).

Test results are shown in Fig. 5. As shown in Fig. 5, similarly to Fig. 4, no change was confirmed in the cell proliferation state of human gingival fibroblasts in growth phase affected by acidic electrolyzed water (AEW), even when DMSO was used. It is thus considered that cells are damaged immediately after treatment with acidic electrolyzed water because of cytotoxicity induced against the cells by ROS levels increased by acidic electrolyzed water, and thus the cells cannot recover from the damage even when ROS is eliminated using DMSO. In contrast, the cell proliferation state was confirmed to be significantly improved when proanthocyanidin was used, suggesting that the cells were repaired and recovered from the damage.

### Test 6: Impact of (+)-catechin on the cytotoxicity of acidic electrolyzed water against mouse fibroblasts in growth phase

Similarly to Test 4, mouse fibroblasts in growth phase were treated with four-fold-diluted acidic electrolyzed water and undiluted acidic electrolyzed water for 30 seconds. After washing with PBS, 100 µl of 1 mg/ml (+)-catechin (Tokyo Chemical Industry Co., Ltd.) prepared with serum-free DMEM was added to each well, followed by 30 minutes of culture under conditions of 37°C and 5%CO₂. After washing with PBS, fresh medium was added, and then the degree of cell proliferation after 24 hours of culture under conditions of 37°C and 5%CO₂ was determined by the MTT method. In addition, a significant difference between groups was tested by Tukey-Krammer's multiple comparison test (multiple comparative analysis).

Test results are shown in Fig. 6. As shown in Fig. 6, when (+)-catechin was used for mouse fibroblasts in growth phase affected by acidic electrolyzed water (AEW), the cell proliferation state was confirmed to be significantly improved. It is thus considered that even cells severely damaged by increased ROS levels immediately after treatment with acidic electrolyzed water can be repaired and recover from damage by the use of (+)-catechin.

### Test 7: Impact of chlorogenic acid, caffeic acid and gallic acid on the cytotoxicity of acidic electrolyzed water against mouse fibroblasts in growth phase

Similarly to Test 4, mouse fibroblasts in growth phase were treated with undiluted acidic electrolyzed water for 30 seconds. After washing with PBS, 100 µl of 1 mg/ml chlorogenic acid (Sigma-Aldrich), caffeic acid (Tokyo Chemical Industry Co., Ltd.) or gallic acid (Tokyo Chemical Industry Co., Ltd.) prepared with serum-free DMEM was added to each well, followed by 5 or 30 minutes of culture under conditions of 37°C and 5%CO₂. After washing with PBS, fresh medium was added, and then the degree of cell proliferation after 24 hours of culture under conditions of 37°C, 5%CO₂ was determined by the MTT method. In addition, a significant difference between groups was tested by Tukey-Krammer's multiple comparison test (multiple comparative analysis).

Test results are shown in Fig. 7. As shown in Fig. 7, when chlorogenic acid, caffeic acid, or gallic acid was used for mouse fibroblasts in growth phase affected by acidic electrolyzed water (AEW), the cell proliferation state was confirmed to be significantly improved. It is thus considered that even cells severely damaged by increased ROS levels immediately after treatment with acidic electrolyzed water can be repaired and recover from the damage by the use of chlorogenic acid, caffeic acid, or gallic acid.

### Test 8: Impact of the time for post-treatment with proanthocyanidin on the cytotoxicity of acidic electrolyzed water against mouse fibroblasts in growth phase

Similarly to Test 4, mouse fibroblasts in growth phase were treated with undiluted acidic electrolyzed water for 30 seconds. After washing with PBS, 100 µl of 1 mg/ml proanthocyanidin prepared with serum-free DMEM was added to each well, and the resultants were each left to sit at room temperature for 1 minute and 5 minutes. After washing with PBS, fresh medium was added, and then the degree of cell proliferation after 24 hours of culture under conditions of 37°C and 5%CO₂ was determined by the MTT method. In addition, a significant difference between groups was tested by Tukey-Krammer's multiple comparison test (multiple comparative analysis).

Test results are shown in Fig. 8. As shown in Fig. 8(a), it was confirmed that the cell proliferation state can be significantly improved by treating cells affected by acidic electrolyzed water (AEW) with proanthocyanidin for 1 minute. Moreover, as shown in Fig. 8(b), it was confirmed that when the cells were treated with proanthocyanidin for 5 minutes, the cell proliferation state was further improved to the cell proliferation state of the control group (control).

### Test 9: Impact of proanthocyanidin on the cytotoxicity of chlorhexidine and iodine against mouse fibroblasts in growth phase

Similarly to Test 4, mouse fibroblasts in growth phase were treated with an undiluted chlorhexidine solution (CHL, Xttrium Laboratories) or an iodine gargle solution 7% (ISO, Meiji Seika Pharma Co., Ltd.) diluted 15-fold with PBS for 30 seconds. After washing with PBS, 100 µl of 1 mg/ml proanthocyanidin prepared with serum-free DMEM was added to each well, and then the resultants were left to stand at room temperature for 5 minutes. After washing with PBS, fresh medium was added, and then the degree of cell proliferation after 24 hours of culture under conditions of 37°C, 5%CO₂ was determined by the MTT method. In addition, a significant difference between groups was tested by Tukey-Krammer's multiple comparison test (multiple comparative analysis).

Test results are shown in Fig. 9. As shown in Fig. 9, when proanthocyanidin (PA) was used for mouse fibroblasts in growth phase affected by chlorhexidine (CHL) or iodine (ISO), the cell proliferation state was confirmed to be significantly improved. It is thus considered that even cells thought to be severely damaged by treatment with a disinfectant such as chlorhexidine or iodine can be repaired and recover from the damage by the use of proanthocyanidin.

### Test 10: Impact of proanthocyanidin on the cytotoxicity of disinfection technique based on photolysis of hydrogen peroxide on human gingival fibroblasts in growth phase

Human gingival fibroblasts in growth phase obtained similarly to Test 5 were washed with PBS, 100 µl of a 500 mM hydrogen peroxide solution was added, and then laser light irradiation with an output of 100 mW (irradiance: 310 mW/cm²) was performed at 405 nm for 30 seconds. Laser light irradiation was performed using a laser apparatus ("RV-1000", RICOH Optical Corporation, currently, RICOH Industrial Solutions Inc.) equipped with indium gallium nitride as a laser diode.

After irradiation, 100 µl of 1000 U/ml catalase (diluted with serum-free DMEM, Wako Pure Chemical Industries, Ltd.) was injected. After completion of this treatment for all wells, washing was performed twice with serum-free DMEM, 100 µl of serum-free DMEM containing 1mg/ml proanthocyanidin was added, and then cells were cultured for 30 minutes. After culture, medium was removed, washing with PBS was performed, 100 µl of fresh medium was added, and then the degree of cell proliferation was determined by the MTT method after 24 hours of culture under conditions of 37°C and 5%CO₂. In addition, a significant difference between groups was tested by Tukey-Krammer's multiple comparison test (multiple comparative analysis).

Test results are shown in Fig. 10. As shown in Fig. 10, the cell proliferation state was also confirmed to be significantly improved, even when proanthocyanidin (PA) was used for human gingival fibroblasts in growth phase, which had been affected by sterilization based on photolysis of hydrogen peroxide (H₂O₂+LD) using hydroxyl radicals as the main contributor to the activity. It is thus considered that even cells thought to be severely damaged by sterilization methods based on the photolysis of hydrogen peroxide can be repaired and recover from the damage by the use of proanthocyanidin.

Each of the above test results reveal the following. Specifically, as shown in Fig. 1 and Fig. 2, acidic electrolyzed water induced cytotoxicity against mouse fibroblasts and human gingival fibroblasts through mediation of increased intracellular ROS levels. The intracellular ROS levels were significantly decreased by treatment with hydroxyl radical scavengers, DMSO and proanthocyanidin, as shown in Fig. 3, suggesting that at least hydroxyl radicals were contained. However, as shown in Fig. 4 and Fig. 5, the cytotoxicity of acidic electrolyzed water against cells in growth phase remained unaffected by DMSO, suggesting that the cells severely damaged by ROS immediately after treatment with acidic electrolyzed water were in a state of being unable to recover from the damage, even when ROS was eliminated thereafter.

However, as shown in Fig. 4 to Fig. 7, cells in such a state significantly recovered from damage when treated with proanthocyanidin, (+)-catechin, caffeic acid, chlorogenic acid, and the like. Moreover, as shown in Fig. 8, in the test with proanthocyanidin, significant recovery was observed even when the time for treatment was as short as 1 minute or 5 minutes. Accordingly, a feature that involves treating a living body with acidic electrolyzed water for disinfection, and then treating it with a polyphenol represented by the above general formula (1) containing proanthocyanidin is a novel, excellent disinfection system capable of significantly improving the condition of tissues damaged by acidic electrolyzed water.

As shown in Fig. 9 and Fig. 10, conditions resulting from disinfectants such as chlorhexidine or iodine, in addition to acidic electrolyzed water, and the cytotoxicity of the sterilization method based on photolysis of hydrogen peroxide using hydroxyl radicals as the main contributor to the activity, were significantly improved by post-treatment with proanthocyanidin. Therefore, a combination of anti-microbial chemotherapy with cytotoxicity and post-treatment with the polyphenol represented by the above general formula (1) is useful as a technology for alleviating damage to tissues caused by anti-microbial chemotherapy.

This is also applicable as a technology for protecting from cytotoxic stimulation that is performed, in the field of regenerative medicine, upon proliferation of various stem cells. For example, bone marrow stem cells require washing treatment; however, cellular damage due to washing treatment can be reduced by adding a small amount of polyphenol during or after the washing treatment.

An example of a disinfection system utilizing the cell repair agent of an embodiment of the present invention is shown in Fig. 11. As shown in Fig. 11, the disinfection system 10 of an embodiment of the present invention is a disinfection system against periodontal infections, which is composed of: an injector body 11 mounted to be able to inject a liquid from an injection port 11a, that is the tip; a disinfection means 12 for injecting acidic electrolyzed water from the injection port 11 a to perform sterilization; and a post-treatment means 13 mounted to be able to inject a polyphenol solution that is the cell repair agent of an embodiment of the present invention from the injection port 11a to a part to be treated by the disinfection means 12.

The disinfection system 10 is used as follows. Specifically, as shown in Fig. 11, draining of an area affected by periodontal disease (periodontal pocket) 1 with acidic electrolyzed water is performed by the disinfection means 12, and then draining of the area affected by periodontal disease 1 with a polyphenol solution is performed by the post-treatment means 13. At this time, cells damaged by the oxidizing power of acidic electrolyzed water can be repaired by post-treatment with the polyphenol solution. In this manner, the disinfection system 10 can safely and efficiently accelerate the sterilization and cure of infected skin or intraoral wounds, for example. Furthermore, the disinfection system 10 is easy to handle and capable of safely and efficiently performing disinfection of infected sites, surgeries, and the like even via short-term treatment.

### Industrial Applicability

Treatment with the polyphenol represented by the above general formula (1) following treatment with acidic electrolyzed water, sterilization based on photolysis of hydrogen peroxide, or anti-microbial chemotherapy using various disinfectants can be used as a medical agent because of its effect. Hence, the disinfection system according to the present invention is useful in the fields of medicine, particularly the fields of skin therapy and endodontic therapy. Moreover, the cell repair agent according to the present invention can be used for repairing cells not only after disinfection with acidic electrolyzed water or the like, but also after a surgery such as a cancer operation, heart operation, transplant surgery, and the like.

### DESCRIPTION OF REFERENCE SIGNS

1: Area affected by periodontal disease
10: Disinfection system
11: Injector body
11 a: Injection port
12: Disinfection means
13: Post-treatment means

## Claims

1. A cell repair agent, containing at least one type of a polyphenol represented by the following general formula (1), a polymer thereof, and a pharmaceutically acceptable salt thereof, 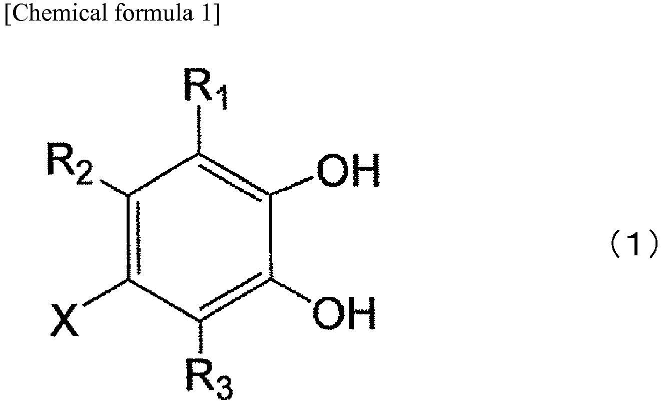 (wherein R₁ to R₃ are each selected from a group consisting of a substituted or unsubstituted hydrogen atom, a hydroxyl group, a halogen atom, a nitro group, a cyano group, a thio group, an alkoxy group, an aryloxy group, a heteroaryloxy group, a carbonyl group, an amino group, a C₁₋₁₀ alkylamino group, a sulfonamide group, an imino group, a sulfonyl group, a sulfinyl group, a C₁₋₁₀ alkyl group, a C₃₋₁₂ cycloalkyl group, a C₃₋₁₂ heterocycloalkyl group, a C₉₋₁₂ bicycloalkyl group, a C₃₋₁₂ heterobicycloalkyl group, a C₁₋₁₀ arylalkyl group, a C₁₋₅ heteroarylalkyl group, a C₁₋₁₀ parhaloalkyl group, a C₁₋₃ carbonylalkyl group, a C₁₋₃ thiocarbonylalkyl group, a C₁₋₃ sulfonylalkyl group, a C₁₋₃ sulfinylalkyl group, a C₁₋₁₀ aminoalkyl group, a C₁₋₃ iminoalkyl group, an aryl group, a heteroaryl group, a C₉₋₁₂ bicycloaryl group, and a C₄₋₁₂ hetero bicycloaryl, are each independent, or, any two of R₁, R₂, and R₃ together form a substituted or unsubstituted ring, and X is a substituted or unsubstituted C₁₋₂₀ aliphatic hydrocarbon group, a C₃₋₂₀ aromatic hydrocarbon group, or a C₃₋₂₀ polycyclic aromatic hydrocarbon group.)

2. The cell repair agent according to claim 1, wherein said R₂ and said R₃ are hydrogen atoms.

3. The cell repair agent according to claim 1 or 2, wherein said R₁ is a hydrogen atom or a hydroxyl group.

4. The cell repair agent according to any one of claims 1 to 3, wherein said polyphenol is selected from a group consisting of proanthocyanidin, (+)-catechin, chlorogenic acid, caffeic acid, and gallic acid.

5. A disinfection system, comprising:
a disinfection means for performing sterilization and/or disinfection for a living body; and
a post-treatment means mounted to be able to bring the cell repair agent of any one of claims 1 to 4 into contact with a part of said living body to be treated by said disinfection means.
